Europäisches Patentamt

European Patent Office

Office européen des brevets

(19)

(11) Veröffentlichungsnummer: **0 031 152**
A2

(12) # EUROPÄISCHE PATENTANMELDUNG

(21) Anmeldenummer: **80108038.3**

(22) Anmeldetag: **19.12.80**

(51) Int. Cl.³: **G 02 C 13/00**
A 61 L 2/04, B 08 B 3/12

(30) Priorität: **19.12.79 DE 2951227**

(43) Veröffentlichungstag der Anmeldung:
**01.07.81 Patentblatt 81 26**

(84) Benannte Vertragsstaaten:
**AT CH FR GB IT LI NL SE**

(71) Anmelder: **Laborgeräte & Medizintechnik Weber GmbH
Waltherstrasse 10
D-8000 München 2(DE)**

(72) Erfinder: **Weber, Helmut
Sepp-Herberger Strasse 21
D-8056 Neufahrn(DE)**

(74) Vertreter: **Riebling, Günter, Dr. Patentanwälte et al,
Dr.-Ing., Dipl.-Ing., Ing.(grad) G. Riebling Dr.-Ing.,
Dipl.-Ing. P. Riebling Rennerle 10 Postfach 3160
D-8990 Lindau(Bodensee)(DE)**

(54) Vorrichtung zur Reinigung von Gebrauchsgegenständen, insbesondere von Kontaktlinsen.

(57) Die Ultraschallreinigungsvorrichtung 1 weist ein im Taschenformat ausgebildetes Gehäuse 8 auf, in dem eine von Ultraschall beaufschlagte, mit einer Flüssigkeit gefüllte Reinigungswanne 27 und ein mit einer anderen Flüssigkeit gefüllter heizbarer Sterilisierungsbehälter 37 angeordnet sind. Die Reinigungswanne 27 und der Sterilisierungsbehälter 37 bestehen jeweils aus zwei voneinander getrennten Wannen 4 - 7, in welche jeweils eine Kontaktlinse 36 mit einem korbartigen Einsatz 28 einsetzbar ist. Die Wannen 4 - 7 werden durch einen gemeinsamen, schwenkbar am Gehäuse 8 angeordneten, flüssigkeitsdichten Verschlussdeckel 26 abgeschlossen.

FIG 1A

FIG 1B

FIG 1C

EP 0 031 152 A2

Vorrichtung zur Reinigung von Gebrauchsgegenständen,
insbesondere von Kontaktlinsen
--------------------------------------------------------

Die Erfindung betrifft eine Vorrichtung zur Reinigung von
Gebrauchsgegenständen, insbesondere von Kontaktlinsen,
mittels eines durch Ultraschall in Schwingungen versetzten
Lösungsbades.

Die Erfindung macht sich die Tatsache zunutze, daß eine Lösungsflüssigkeit mit Hilfe von Ultraschallquellen in Schwingungen versetzt werden kann, wobei in der Lösungsflüssigkeit enthaltene Gegenstände durch die mechanischen Reibungskräfte zwischen der Lösungsflüssigkeit und den Gegenständen von Schmutzablagerungen befreit werden. Der Vorteil
dieses Verfahrens besteht darin, daß sehr kleine Geräte und
Bauelemente in kurzer Zeit von Verunreinigungen befreit
werden können, was mit mechanischen Bürsten oder dergleichen
nicht möglich wäre. So lässt sich zum Beispiel die erfindungsgemässe Reinigungsvorrichtung ausgezeichnet in Laboratorien zur Reinigung von Laborglas, Laborgeräten, Mikropräzisionssieben, Küvetten, Brillen, Schmuck, Münzen, Uhren,
Hörgeräten, Pinzetten, Instrumenten, Sonden, Halbleitern,
Magnetköpfen, Relais, Optiken usw. einsetzen. In bestimmten
Anwendungsfällen, zum Beispiel zum Reinigen von Brillengläsern und Kontaktlinsen, wäre es wünschenswert, wenn eine
Reinigungsvorrichtung zur Verfügung stünde, welche der Benutzer ohne Beeinträchtigung der Bequemlichkeit und Verminderung der Sicherheit durch Auslaufen von Lösungsflüssigkeit und dergleichen bei sich tragen könnte, um sie im Bedarfsfalle sofort einzusetzen.

Die Erfindung ist deshalb darauf gerichtet, eine Reinigungsvorrichtung der oben beschriebenen Art zu schaffen, welche
platzsparend, bequem in der Handhabung und betriebssicher ist, sowie dem Benutzer jederzeit gebrauchsfertig zur Verfügung steht.

Gemäss der Erfindung wird eine Reinigungsvorrichtung der im Oberbegriff des hauptanspruchs beschriebenen Art vorgeschlagen, die eine Energiequelle, z.B. netzabhängig, zur Speisung der Einrichtung zur Erzeugung der Schwingungen des Lösungsbades aufweist und bei welcher das Lösungsbad, die Energiequelle und die Schwingungseinrichtung in einem flüssigkeitsdichten Kleinbehälter angeordnet sind.

Weitere vorteilhafte Ausgestaltungen des Gegenstandes der Erfindung ergeben sich aus den Unteransprüchen.

Gemäss der Erfindung wird eine kleine, handliche Reinigungsvorrichtung vorgeschlagen, welche der Benutzer zum Beispiel im Jackett stets bei sich tragen kann, wobei eine geeignete Verschlusseinrichtung dafür sorgt, dass das Lösungsbad in einer flüssigkeitsdichten Wanne aufgenommen ist, so dass das Gerät während des Transportes eingeschaltet werden kann und den Reinigungsvorgang durchführt, ohne dass für den Träger des Gerätes ein Sicherheitsrisiko auftritt. Der Schwingungsgeber schaltet sich nach einer bestimmten Reinigungszeit automatisch ab, wobei diese Reinigungszeit in Abhängigkeit von der Anzahl der zu reinigenden Gegenstände vorgewählt werden kann. Es wäre allerdings auch denkbar, dass sich die Reinigungszeit in Abhängigkeit von dem Flüssigkeitsstand einstellt, der wiederum in Abhängigkeit der Anzahl der zu reinigenden Geräte höher oder niedriger sein kann.

Bei den Schwingungsgebern handelt es sich vorteilhaft um im Rechteck schwingende Oszillatoren, wobei die Schwingungen derart erzeugt werden, dass viele harmonische Schwingungen auftreten. Hierzu sind die Schwingungsgeber auf die Resonanzfrequenz der Reinigungswanne eingestellt.

Als Energieträger finden vorzugsweise Primär- bzw. Sekundärelemente Verwendung.

Darüberhinaus ist die Erfindung darauf gerichtet, den Lösungsbadteil der Reinigungsvorrichtung derart zu gestalten, daß in dem Bad aufgenommene Kontaktlinsen bei einer in beliebiger Lage am Körper getragenen und sich in Funktion befindlichen Vorrichtungen während der Behandlung keinen schädlichen Einflüssen, z.B. durch einen unmittelbaren Kontakt mit der schwingenden Wanne ausgesetzt sind, die zu einer Zerstörung oder Beschädigung der Kontaktlinsen führen könnten.

Die erfindungsgemässe Vorrichtung besteht aus einem taschenformatförmigen Kleinbehälter, in welchem die zur Aufnahme des Lösungsbades und mittels eines Schwingungsgebers in Schwingungen versetzbare Reinigungswanne flüssigkeitsdicht angeordnet ist. Die flüssigkeitsdichte Abdichtung der Reinigungswanne erfolgt mittels eines Korbeinsatzes, der einen umlaufenden Randwulst besitzt, mit dem der Korbeinsatz auf einem umgebördelten Rand der Reinigungswanne aufliegt und gegen welchen ein aufklappbarer Gehäusedeckel des Kleinbehälters im Schließzustand unter Druck anliegt. Dieser Einsatz besitzt zwei Körbe, die freitragend in die Reinigungswanne hineinhängen und vom Lösungsbad umspült sind. Zur Verringerung der Masse besitzt der Einsatz Schlitze und Bohrungen, die gleichzeitig den Durchtritt des Lösungsbades ermöglichen. Die Körbe des Einsatzes sind derart dimensioniert, daß jeweils eine Kontaktlinse darin aufgenommen werden kann. Im Bodenbereich läuft jeder Korb vorteilhaft konisch zu, so daß sich bei Aufliegen der Kontaktlinse eine ringförmige, stabile Abstützung ergibt.

Weitere Merkmale, Einzelheiten und Vorteile der Erfindung

- 4 -

ergeben sich aus der nachfolgenden Beschreibung eines bevorzugten Ausführungsbeispiels an Hand der Zeichnung. Darin zeigen:

Fig. 1a eine Draufsicht auf die erfindungsgemässe Ultraschallreinigungsvorrichtung;

Fig. 1b Draufsicht auf das Gerät mit aufgeklapptem Verschlussdeckel;

Fig. 1c Ansicht des Schalt- und Bedienungsteiles;

Fig. 2 eine Teilschnittansicht der erfindungsgemässen Vorrichtung entlang der Linie I-I nach Fig. 3,

Fig. 3 eine Draufsicht auf einen Teil der Vorrichtung;

Fig. 4 eine schematische Teilschnittansicht entlang der Linie III-III nach Fig. 2;

Fig. 5 ein Blockschaltbild der elektronischen Schaltung für die Einrichtung zur Erzeugung der Schwingungen des Lösungsbades.

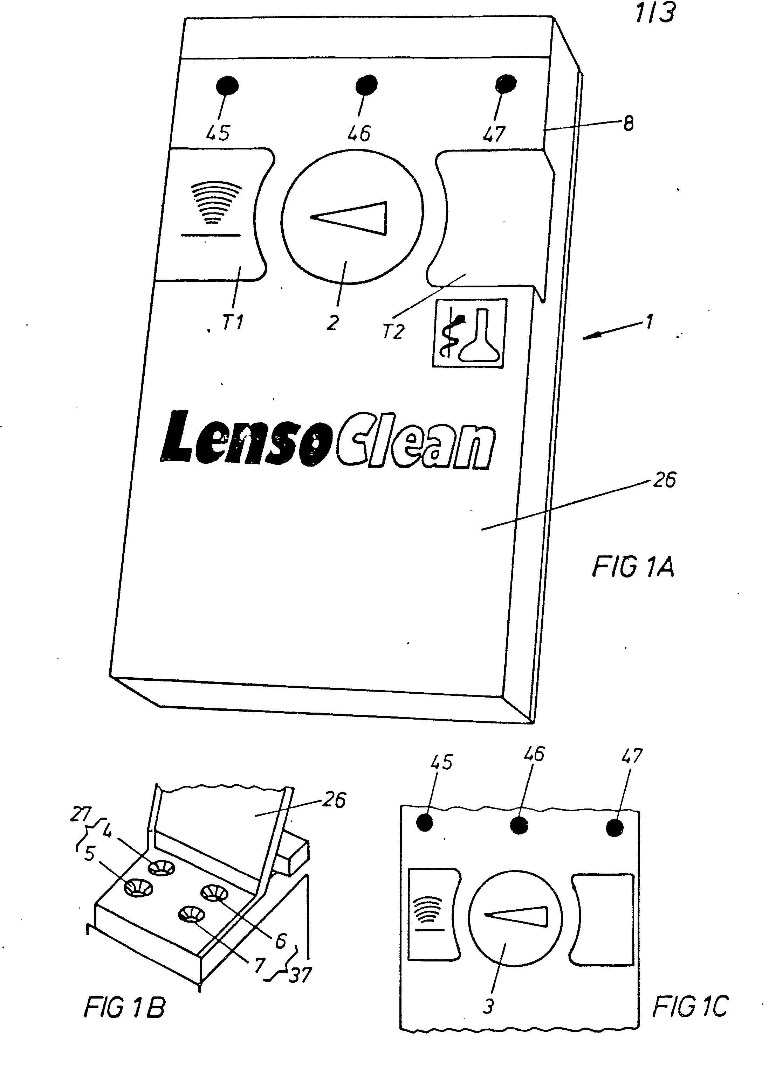

An Hand der Fig. 1 soll die Inbetriebnahme des Gerätes erläutert werden.

1. Das Steckernetzgerät in die Steckdose, die Buchse am 2-adrigen Niederspannungskabel in den Gerätestecker hinten am Gerät einstecken. Die gelbe Leuchtdiode 47 brennt und zeigt die Betriebsbereitschaft des Gerätes an.

2. Den Klappdeckel 26 öffnen, die Wannen 4 - 7 mit Flüssigkeit füllen. Der linke Behälter 4, 5 unterhalb des Schalters 6 ist der Ultraschall-Reinigungsbehälter 27. Der Behälter 4, 5 wird mit ca. 5 - 6 cm (Kubikzentimeter) Reinigungsflüssigkeit gefüllt, das entspricht etwa einer 2/3 Füllung des Behälters.

3. Der rechte Behälter 6,7 unterhalb des Schalters 6 ist der Sterilisierungsbehälter 37. Dieser Behälter 6,7 wird ebenfalls zu 2/3 mit einer Reinigungs-Abspül- und Aufbewahrungsflüssigkeit aufgefüllt.

4. Die Kontaktlinsen in den Tauchkorb legen. Der Tauchkorb ist mit zwei Buchstaben R und L, für die rechte und linke Kontaktlinse gekennzeichnet. Den Tauchkorb mit den Kontaktlinsen in den linken Behälter 4,5 (Ultraschallschwingwanne) stellen.

5. Den Schalter 2 am Gerät zur Taste T1 drehen, dadurch wird die mechanische Verriegelung der Taste T1 aufgehoben. Durch Druck auf die T1-Taste wird der Ultraschall eingeschaltet, die rote Leuchtdiode 45 über der Drucktaste brennt - die gelbe Leuchtdiode 47 erlischt. Durch einen elektronisch gesteuerten Zeitkreis wird der Ultraschall nach 1o Minuten automatisch ausgeschaltet, die gelbe Leuchtdiode 47 brennt wieder und die rote Leuchtdiode 45 erlischt.

6. Für harte Kontaktlinsen ist somit der Reinigungsprozess abgeschlossen. Die Linsen werden jetzt noch mit Abspül- und Aufbewahrungsflüssigkeit behandelt und wieder in die Aufbewahrungsbehältnisse zurückgegeben.

7. Die weichen Kontaktlinsen werden weiterbehandelt, es erfolgt noch eine Hitzesterilisation. Der Tauchkorb mit den Kontaktlinsen wird aus dem Ultraschallbehälter 4,5 herausgenommen und in den rechten gefüllten Behälter 6,7 (Sterilisierungswanne) gestellt. Den Zeigerknopf (Schalter 2) am Gerät nach rechts drehen zur T2-Taste. Die mechanische Verriegelung der Drucktaste ist aufgehoben. Durch Druck auf die T2-Taste wird jetzt die Heizung eingeschaltet. Die rote Leuchtdiode 46 über der Drucktaste und die gelbe Leuchtdiode 47 brennen gemeinsam. Die gelbe Leuchtdiode 47 brennt solange mit, bis die Badtemperatur erreicht ist, dann schaltet sich der elektronische Zeitkreis ein, der die Badtemperatur 10 Minuten konstant hält und anschliessend die Heizung wieder ausschaltet. Jetzt erlischt die rote Leuchtdiode 46 und die gelbe Leuchtdiode 47 blinkt. Das Blinken hält solange an, bis die Flüssigkeit auf eine Temperatur von ca. 35° - 4o° Cels. abgekühlt ist. Der Tauchkorb mit den Kontaktlinsen verbleibt bis zum Gebrauch derselben in dem mit Reinigungs-, Abspül- und Aufbewahrungsflüssigkeit gefüllten Behälter 6,7. Der Tauchkorb mit den Kontaktlinsen kann jedoch auch direkt nach dem. Erlöschen der roten Leuchtdiode 46 dem Behälter 6,7 entnommen werden, dabei ist Vorsicht geboten wegen VERBRÜHUNGS-GEFAHR!

WICHTIG: Beim Sterilisieren Behälter mit dem Klappdeckel 3 verschliessen.

8. Verbrauchte Lösungen können mit dem mitgelieferten Schwamm aus den Behältern abgesaugt werden. Der Schwamm ist vor Gebrauch anzufeuchten und hochkant in den Behälter langsam einzudrücken. Den Schwamm anschließend gut unter fliessendem Wasser ausspülen.

Die in der Fig. 1 gezeigte Ultraschallreinigungsvorrichtung 1 besteht im wesentlichen aus einem Gehäuse 8, welches in etwa Taschenformat besitzt und in welchem die Einrichtung zur Erzeugung der Schwingungen des Lösungsbades sowie die Energiequelle für die Schwingungseinrichtung aufgenommen sind.

Bei dem dargestellten Ausführungsbeispiel ist die Energiequelle von vier netzunabhängigen, elektrochemischen Elementen, nämlich wiederaufladbaren NC-Akkumulatoren gebildet, so daß das Gerät unabhängig von dem Netzstrom mitgenommen und in Betrieb genommen werden kann. Es wäre denkbar, einen Netzanschluss vorzusehen, der das Aufladen der sich in dem Gehäuse befindlichen NC-Akkumulatoren ermöglicht, so daß diese Akkumulatoren zum Aufladen nicht aus dem Gehäuse herausgenommen werden müssen.

Die Akkumulatoren können auch als Energiespeicher für Solarzellen dienen, die auf der Oberseite des Gehäuses, zum Beispiel zwischen dem Verschlußdeckel 26 für die Reinigungswannen4 - 7 angeordnet sein könnten. Diese Solarzellen sind in der Zeichnung nicht dargestellt.

Bei den Bedienungselementen handelt es sich um Tasten T1 und T2, die bei Berührung das Gerät einschalten und bei einer nochmaligen Berührung dafür sorgen, daß die Zeitautomatik auf den Ausgangswert Null zurückgestellt wird. Erfolgt keine zweite Berührung, so stellt die Zeitautomatik die Vorrichtung nach einer bestimmten Reinigungszeit ab.

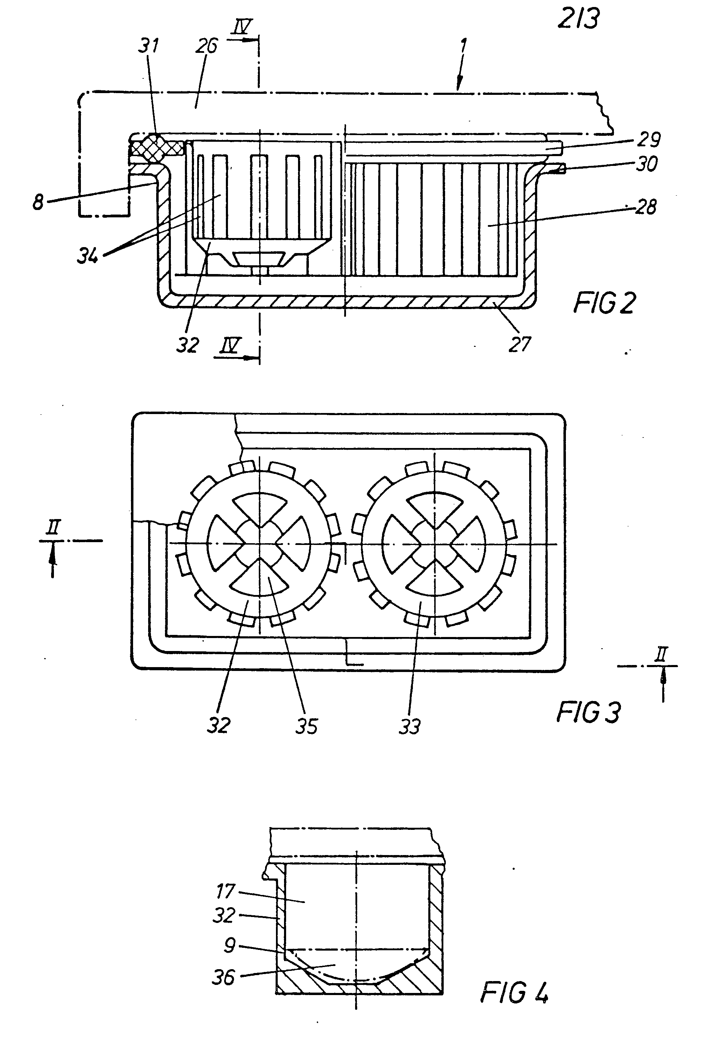

Die Reinigungswanne 27, die zur Aufnahme des Lösungsbades dient, besteht aus V2A-Stahl oder einem 18/8-Chromnickelstahl. In der Reinigungswanne befindet sich ein Einsatz 28, der in der Fig. 3 gestrichelt dargestellt ist und in seiner Höhe derart bemessen ist, daß er eine strichpunktiert dargestellte Kontaktlinse 36 mit ihrem Rand 9 gegen die Unterseite des Verschlußdeckels 26 drückt. Die Kontaktlinse 36 wird dadurch in ihrer Lage innerhalb des Lösungsbades fixiert, ist jedoch von dem Lösungsbad 17 vollständig umspült. Die erfindungsgemässe Ultraschallreinigungsvorrichtung 1 arbeitet daher unabhängig von der jeweiligen Gebrauchslage zuverlässig.

Aus den Fig. 2 und 3 ist zu entnehmen, daß der Verschlußdeckel 26 den Rand des Gehäuses 8 umgreift und mittels einer nicht dargestellten Arretierungslasche in eine Gehäuseausnehmung eingreift. Hierdurch wird gewährleistet, daß sich der Deckel nicht ungewollt öffnen kann, was insofern

-8-

wichtig ist, da das Gerät in allen denkbaren Stellungen betriebsbereit sein muss und gewährleistet sein muss, daß keine Reinigungsflüssigkeit in diesen Lagen austreten kann.

In der Fig. 2 und 3 ist im wesentlichen nur der Reinigungswannenteil der erfindungsgemässen Ultraschallreinigungsvorrichtung 1 dargestellt. Die Reinigungswanne 27 nimmt einen Einsatz 28 auf, der mit einem Randabschnitt 29 auf einem umgebördelten Rand 30 der Reinigungswanne aufliegt.

Der Randabschnitt 29 des Einsatzes 28 besitzt einen umlaufenden Wulst 31, gegen den der Verschlußdeckel 26 der Ultraschallreinigungsvorrichtung unter Druck anlegbar ist, so daß der Einsatz 28 arretiert und die Reinigungswanne 27 flüssigkeitsdicht verschlossen ist.

Der Einsatz 28 besitzt zwei Körbe 32,33, die zur Aufnahme von Kontaktlinsen bestimmt sind. Die Wandungen dieser Körbe bzw. die Wandung des Einsatzes weisen Schlitze 34 bzw. Durchbrechungen 35 auf, so daß das Lösungsbad in die Körbe eindringen kann und gleichzeitig das Gewicht des Einsatzes und damit die schwingende Masse verringert wird. Der Einsatz und die Körbe bestehen aus einem einstückigen Kunststoffgußteil und können z.B. gitterartig aufgebaut sein. Da die Berührung zwischen der schwingenden Wanne 27 und den Körben 32,33 nur über den Ringwulst 31 erfolgt, befinden sich die Körbe im wesentlichen in Ruhe, so daß die Schwingungen von der Wanne im wesentlichen nur in das Lösungsbad eingeleitet werden.

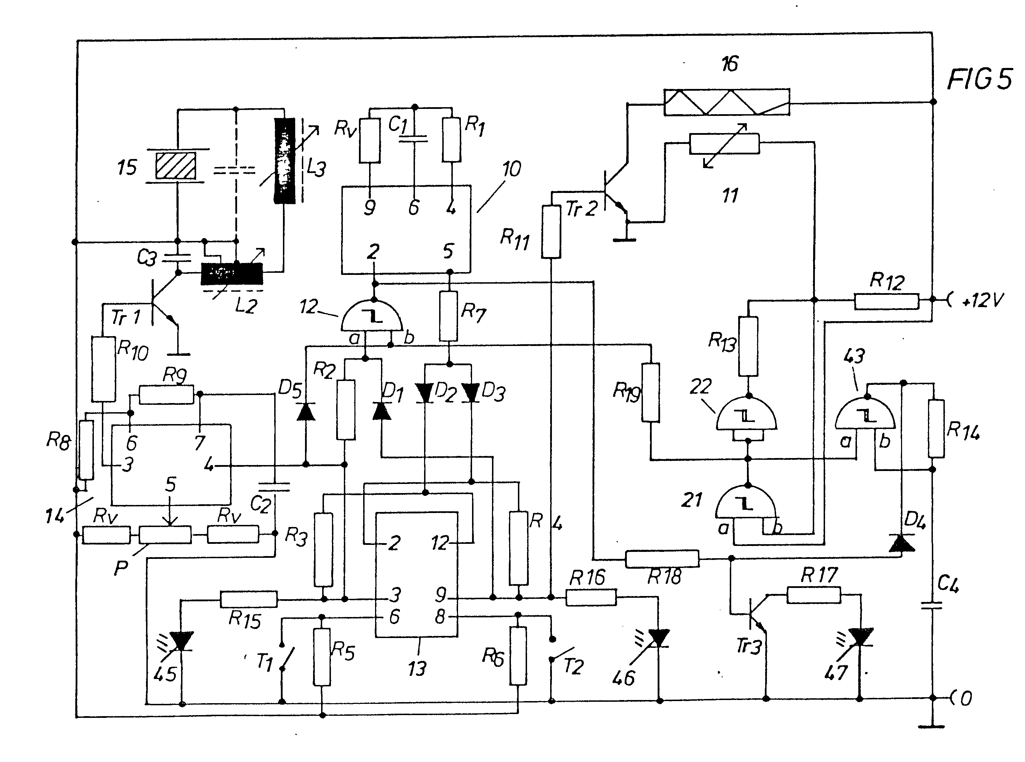

Aus Fig. 4 ist zu entnehmen, daß Körbe im Bodenbereich konisch verlaufen, so daß eine Kontaktlinse 36 im wesentlichen entlang einer ringförmigen Linie auf dem Boden eines Korbes abgestützt wird.

Die elektronische Steuerung besteht im Prinzip aus drei Teilen, die sinngemäss zusammengeschaltet sind, wie weiter unten beschrieben ist.
Die Teile sind:

1. ein Zeitgeber, der nach Ablauf von 10 Minuten ein positives Signal abgibt.

2. Eine mit den Start-Tasten verbundene Verriegelungsschaltung, die gewährleistet, dass ein nochmaliger Druck auf dieselbe Taste oder auch gegebenenfalls nach Lösung der mechanischen Verriegelung auf die andere Taste keine Störung des ablaufenden Programms auslöst.

3. Einen Zweipunkt-Temperaturregler, der dafür sorgt, dass die Zeitstrecke von 10 Minuten erst dann beginnt, wenn die Wannentemperatur bei der thermischen Sterilisation $80^{\circ}$ C erreicht hat und nach Programmablauf solange eine Blinkschaltung betätigt, bis eine Wannentemperatur beim Abkühlvorgang von $40^{\circ}$ C unterschritten wird.

Fig. 5 zeigt die Schaltungsanordnung der elektronischen Ablaufsteuerung, aus der weitere wesentliche erfinderische Merkmale hervorgehen.

Als Zeitgeber 10 wird ein 23stufiger Binäruntersetzer verwendet, der mittels R-C-Generator angesteuert wird. In dem Baustein z.B. MC 14521 sind sowohl die aktiven Elemente für den Generator als auch der Untersetzer integriert. Seine Anschlüsse P in 6 und Pin 4 sind in bekannter Weise mit den frequenzbestimmenden Gliedern $C_1$ und $R_1$ beschaltet, so dass die Ansteuerfrequenz ca. 7 Khz beträgt. Die nach 23facher Untersetzung an Pin 15 abnehmbare Halbperiode nimmt dann nach 10 Minuten H-Potential an. Die Laufzeit errechnet sich aus dem Kehrwert des Quotienten aus Frequenz durch Teilerstufen. Der Rückstelleingang Pin 2 ist mit dem Ausgang des NAND-Gatters 12 verbunden, welches mit seinem Eingang a über

- 10 -

den Widerstand $R_2$ und die Diode $D_1$ an die Ausgänge der Verriegelungsschaltung 13 angeschlossen ist. An 13 ist jeweils der Ausgang H-Potential, dessen Eingang durch den Schalter 2 mit den Schaltstellungen $T_1$ (Ultraschall) oder $T_2$ (Heizung) getriggert wurde. Der Eingang b des Gatters 12 ist mit dem Widerstand 19 an den Temperaturregelkreis und mittels der Diode $D_5$ an den Ausgang Pin 3 der Verriegelungsschaltung 13 angeschlossen, so dass das Gatter 12 in der angegebenen Schaltung die ·- zum potential-richtigen Betrieb des Rückstelleingangs Pin 2 10 erforderliche NOR-Funktion ausführt.

Als Baustein für die Verriegelungsschaltung 13 wird eine Doppelzeitgeberschaltung z.B. LM 556 verwendet, die zwei getrennte gleichartige Schaltkreise enthält. Abweichend von bekannten Applikationen werden beide Schaltkreise ohne Ladekondensator betrieb en, sondern die Komparatoreingänge Pin 2 und Pin 12 über Koppel- widerstände $R_3$ und $R_4$ so an die Ausgänge Pin 3 und Pin 9 angeschlossen, dass jeweils der Komparator des einen Teils mit dem Ausgang des anderen verbunden ist. Dadurch ergibt sich die Verriegelungsschaltung, weil H-Potential am Komparatoreingang zu L-Potential am Ausgang führt und ausserdem eine Triggerung unterbindet.

Die mit den Tasten $T_1$ bzw. $T_2$ verbundenen Triggerein- gänge sind über die Widerstände $R_5$ und $R_6$ nach H-Poten- tial gezogen, damit im Einschaltmoment der Betriebsspannung beide Hälften der Verriegelungsschaltung in Ausgangsstellung, also mit L-Potential an den Ausgängen stehen.

Die beiden Komparatoreingänge sind weiter über die Dioden $D_2$ und $D_3$ und dem gemeinsamen Widerstand $R_7$

an den Ausgang Pin 15/1 gelegt, so dass bei H-Potential an Pin 15 der jeweils getriggerte Teil der Verriegelungsschaltung 13 wieder in Anfangsstellung gebracht ist.

Am Ausgang Pin 3/3 (Steuerausgang für den Ultraschallreinigungsvorgang) ist der Rechteckgenerator 14 angeschlossen, der z.B. mit dem integrierten Baustein LM 555 aufgebaut ist. An diesen sind in bekannter Weise an Pin 6 und 7 die frequenzbestimmenden Glieder $R_8$, $R_9$. und $C_2$ angeschlossen, so dass der Generator mit ca. 35 Khz schwingt. Diese Mitten-Frequenz lässt sich durch $P_1$ um einen ausreichenden Betrag verstellen, so dass diese an die mechanische Resonanz der aus Ultraschallwanne und Schwinger bestehenden Einheit angeglichen werden kann. Der Ausgang des Generators 14 ist über den Vorwiederstand $R_{10}$ mit der Basis des Leistungstransistors $Tr_1$ verbunden, der mit seiner weiteren Beschaltung die Ultraschall-Endstufe darstellt.

Der Kollektor des Transistors $Tr_1$ ist mit der Spule $N_1$ und dem Kondensator $C_3$ verbunden. $L_1$ ist mit $C_3$ auf die Mittenfrequenz des Steuergenerators 14 auf 35 Khz abgestimmt und stellt den sogenannten Tank-Kreis dar. Dieser Kreis ist mit verhältnismässig hohem C und kleiner Selbstinduktion von $L_1$ ausgeführt, damit sich eine breite Resonanz ergibt. Die auf demselben Kern aufgebrachte Zusatzwicklung $L_2$ dient der Anpassung des aus der Spule $L_3$ und der Eigenkapazität des Schwinges 15 gebildeten Serienresonanzkreises. Der gestrichelt eingezeichnete Kondensator soll die Eigenkapazität des Schwingers darstellen. Bei Resonanz, die sich durch Veränderung der Selbstinduktion von $L_3$ einstellen lässt, ergibt sich am Ende von $L_3$, welches mit dem "heissen" Anschluss des Schwingers verbunden ist, die zum Betrieb des Schwingers erforderliche Spannungserhöhung auf ca.

400 Volt. $L_3$ lässt ausserdem eine stufenlose Leistungsregelung zu, damit die Ultraschallintensität eingestellt werden kann. Die Serienresonanz-Schaltung hat weiter den Vorteil, dass bei schadhaft werden des Schwingers oder z.B. Brechen der Anschlussdrähte die Serienresonanz zusammenbricht, damit keine Leistung mehr aus dem Tankkreis entzogen und der Transistor $Tr_1$ nicht überlastet werden kann.

An Pin 9/3, der den Steuerausgang für die Heizung bei der thermischen Sterilisation darstellt und durch $T_2$ getriggert wird, ist über den Vorwiderstand $R_{11}$ der Leistungstransistor $Tr_2$ angeschlossen, der im Kollektorkreis den Heizwendel 16 enthält. Dieser Transistor arbeitet als Schalter. Der Heizwendel ist auf dem Boden der Sterilisationswanne angebracht. Am Wannenrand befindet sich der Temperaturfühler 11, der z.B. mit einem NTC-Widerstand ausgestattet ist. Er dient als Temperaturaufnehmer für die Zweipunkt-Regelung.

Die Gatter 12, 21, 22 und 23 sind Schmitt-Trigger-Nand-Gatter, so dass diese als Komparator für die Temperaturregelung verwendet werden. Der ingetrierte Baustein ist z.B. MC 14093, der vier Gatter mit je zwei Eingängen enthält.

Die Gatter 21 und 22 bilden mit ihrer äusseren Beschaltung den Zwiepunkt-Temperaturregler. Der Eingang a/11 liegt an H-Potential, so dass das Gatter nur über den Eingang b gesteuert wird. Letzterer ist mit dem Widerstand $R_{12}$, der nach + führt, dem Widerstand $R_{13}$, der am Ausgang des Gatters 22 angeschlossen ist, sowie mit dem Temperaturfühler 11 verbunden, dessen anderes Ende an O-Potential liegt. Der Fühler 11 bildet mit dem Widerstand $R_{12}$ die Regelbrücke, so dass durch Wahl des Wider-

standswertes von $R_{12}$ die obere Triggerschwelle von 11 festgelegt werden kann, die auf 80° C festgelegt ist. Bei niederer Temperatur (Einschaltzeitpunkt des Gerätes) sind beide Eingänge von 21 an H getriggert, so dass am Ausgang von 21 L-Potential ansteht. Der Ausgang von 21 ist über den Widerstand $R_{19}$ mit dem Eingang b von 12 verbunden, so dass der Zeitgeber 10 gesperrt bleibt. Sobald die Regelbrücke den oberen Schwellwert erreicht, wird der der Ausgang von 21 H-Potential und der Zeitgeber läuft an. Mit dem Ausgang von 21 ist einmal der Blinkgenerator 43 verbunden, der seine Tätigkeit solange beibehält, bis nach Abkühlen der Sterilisations-Wanne der Ausgang des Gatters 21 wieder L-Potential annimmt.

Am Ausgang von 21 ist ausserdem das Gatter 22 als Inverter angeschlossen, das je nach logisch L oder H den Widerstand $R_{13}$ parallel zu $R_{12}$ oder dem NTC-Widerstand 11 legt. Bei Anfangstemperatur (H-Potential an den Eingängen von 21) hat auch der Inverterausgang 22 H-Potential, der Widerstand $R_{13}$ ist folglich parallel zur $R_{12}$ geschaltet, so dass der NTC-Widerstand einen kleineren Wert annehmen muss, um 11 zu triggern. Nach Erreichen dieser Temperatur ist der Ausgang des Inverters logisch L, so dass nunmehr der NTC-Widerstand einen höheren Wert annehmen, also weiter abgekühlt sein muss, um die Schaltung wieder in den Ausgangszustand zu bringen. Die Grösse des Widerstandes $R_{13}$ bestimmt somit, wie weit Einschalt- und Ausschaltschwelle auseinanderliegen. Bei der vorliegenden Schaltung liegt die Einschaltschwelle bei 80° C und die Ausschaltschwelle bei 40° C. Dabei haben die Widerstände folgenden Wert: $R_{12}$ = 39 K, R 13 = 120 k NTC-Widerstand 100 K Kaltwiderstand.

Am Ausgang 3 von 13 ist über den Widerstand $R_{15}$ die Anzeigediode 45 angeschaltet, so dass diese die erfolgte Triggerung des Ultraschall-Reinigungskreises anzeigt. Am Ausgang Pin 9 von 13 ist in gleicher Weise die Anzeigediode 46 angeschlossen, die die erfolgte Triggerung des Heizkreises anzeigt. Die Anzeigediode 47, die mit ihrem Vorwiderstand $R_{17}$ am Kollektor des Transistors $TR_3$ angeschlossen ist, leuchtet immer dann, wenn der Zeitkreis ausgeschaltet ist, der Ausgang des Gatters 2 positives Potential führt. Der Basiswiderstand von $Tr_3$ liegt an diesem Ausgang. Ausserdem ist die Basis von $Tr_3$ über die Diode $D_4$ mit dem Ausgang des Blinkgenerators 43 verbunden, so dass bei H-Potential am Gatter 12 die Ansteuerung an der Basis von $Tr_3$ im Takt des Blinkgenerators unterbrochen wird, sofern die Wannentemperatur noch nicht unter 40° C abgesunken ist.

Der zeitliche Funktionsablauf der Steuerung kann nun wie folgt beschrieben werden:

1. Ultraschallreinigung:

Nach Triggerung der Verriegelungsschaltung 13 durch Taste $T_1$ nimmt der Ausgang Pin 3 positives Potential an. Mit diesem Potential wird der Frequenzgenerator und damit die Ultraschallendstufe in Tätigkeit gesetzt. Gleichzeitig erhalten die Gattereingänge von 12 einmal über die Diode $D_5$ und dann über den Widerstand $R_2$ positives Potential, so dass der Zeitkreis startet. Gleichzeitig wird die an Pin 3 von 13 liegende Anzeigediode 45 über $R_{15}$ angesteuert und leuchtet auf, während bedingungsgemäss die Anzeigediode 47, die vor der Triggerung durch $T_1$ den betriebsbereiten Zustand angezeigt hat, erlischt.

Nach Ablauf des Zeitgebers 10 wird über dessen Ausgang Pin 15 der Widerstand $R_7$ und die Diode $D_3$ die Verriegelungsschaltung 13 wieder auf Anfangsstellung gebracht .

Der Ausgang Pin 3 von 13 nimmt wieder L-Potential an, so dass die Anzeigediode 45 erlischt und die Anzeigediode 47 das Ende des Programms anzeigt.

2. Thermische Sterilisation:

Nach Triggerung durch $T_2$ nimmt der zugehörige Ausgang Pin 9 H-Potential an. Die zugehörige Anzeigediode 46 leuchtet auf und zeigt an, dass das Programm gestartet ist. Die Anzeigediode 47 bleibt zunächst in Funktion, weil der Gattereingang von 12 über den Ausgang des Gatters 21 und $R_{19}$ noch solange L-Potential führt, bis durch den Regelkreis Gatter 21 umgeschaltet wird. In diesem Augenblick - Erreichen der Sterilisations-Temperatur ($80^{\circ}$ C) - wird der Ausgang des Gatters 21 H-Potential, startet damit den Zeitkreis und die Anzeigediode 47 erlischt, zum Zeichen, dass die Sterilisationstemperatur erreicht ist und der eigentliche Sterilisationsvorgang beginnt.

Nach Ablauf des Zeitkreises wird die Verriegelungsschaltung wieder in Anfangstellung gebracht, dadurch, dass über Pin 15 (10) H-Potential gelegt wird, und zwar über $R_7$ und $D_2$ an den Komparatoreingang Pin 12 (10). Jetzt erlischt die Anzeigediode 46 zum Zeichen, dass der Sterilisationsvorgang beendet ist. Gleichzeitig steuert das am Ausgang von Gatter 12 liegende H-Potential den Transistor $Tr_3$ an, so dass die Anzeigediode 47 wieder aufleuchtet. Da die Basisspannung von $Tr_3$ jedoch über die Diode $D_4$ an den Blinkgenerator 43 angeschaltet ist, gibt die Anzeigediode solange ein Blinkzeichen, bis die Wannentemperatur $40^{\circ}$ C unterschritten hat. Dann geht das Blinken in ein Dauerleuchten über und zeigt an, dass das Gerät nun für eine weitere Ultraschallreinigung oder Sterilisation betriebsbereit ist.

Die Schaltung kann ohne Schwierigkeiten auf dem zur Verfügung stehenden Platz, einer quadratischen Fläche von ca. 7 cm Kantenlänge, einschliesslich der Triggertasten bei einseitiger Kaschierung der Basisplatte untergebracht werden, so dass die Herstellungskosten günstig sind. Günstig zu bewerten ist auch die Sicherheit gegen Bedienungsfehler, die die elektronische Steuerung aufweist.

Der Vorteil für den Anwender besteht hauptsächlich in Zeitersparnis, Ersparnis an Reinigungsflüssigkeit und wesentlicher Verlängerung der Lebensdauer bei verhältnismässig teueren Kontaktlinsen.

P a t e n t a n s p r ü c h e

---

1. Vorrichtung zur Reinigung von Gebrauchsgegenständen, insbesondere von Kontaktlinsen, mittels eines durch Ultraschall in Schwingungen versetzten Lösungsbades, d a d u r c h  g e k e n n z e i c h n e t , dass eine Energiequelle, z.B. netzabhängig, zur Speisung der Einrichtung zur Erzeugung der Schwingungen des Lösungsbades vorgesehen ist und dass das Lösungsbad, die Energiequelle und die Schwingungseinrichtung in einem flüssigkeitsdichten Kleinbehälter angeordnet sind.

2. Vorrichtung zur Reinigung von Gebrauchsgegenständen, insbesondere von Kontaktlinsen, mittels eines durch Ultraschall in Schwingungen versetzten Lösungsbades nach Anspruch 1, d a d u r c h  g e k e n n z e i c h n e t , dass ein taschenformatförmiger Kleinbehälter vorgesehen ist, in welchem eine flüssigkeitsdicht verschliessbare, in Schwingungen versetzbare Reinigungswanne (27) zur Aufnahme des Lösungsbades angeordnet ist sowie ein zur Hitzesterilisation vorgesehener Sterilisierungsbehälter (37).

3. Vorrichtung nach Anspruch 1, d a d u r c h  g e k e n n z e i c h n e t , dass die Reinigungswanne (27) und der STerilisierungsbheälter (37) mit einem aufklappbaren Gehäusedeckel (26) des Kleinbehälters verschließbar sind.

4. Vorrichtung nach Anspruch 1 oder 2, d a d u r c h  g e k e n n z e i c h n e t , dass in der Reinigungswanne (27) ein korbartiger Einsatz (28) angeordnet ist, welcher freihängend in das Lösungsbad eintaucht.

5. Vorrichtung nach einem der Ansprüche 1 bis 4, d a d u r c h g e k e n n z e i c h n e t , dass die Reinigungswanne (27) einen umgebördelten Rand (30) aufweist und dass der Einsatz (28) mit einem Randabschnitt (29) auf dem Rand (30) der Reinigungswanne (27) aufliegt.

6. Vorrichtung nach einem der Ansprüche 1 bis 5, d a d u r c h g e k e n n z e i c h n e t , dass die Dichtung zwischen der Reinigungswanne (27) und dem Gehäusedeckel (26) von einem umlaufenden Randwulst (31) des Einsatzes (28) gebildet ist.

7. Vorrichtung nach einem der Ansprüche 4 bis 6, d a d u r c h g e k e n n z e i c h n e t , dass der Einsatz (28) wenigstens zwei Körbe (32, 33) zur Aufnahme je einer Xontaktlinse (36) aufweist, die mit Schlitzen (34), Durchbrechungen (35) und dergleichen versehen sind, um ein Durchtreten des Lösungsbades zu ermöglichen.

8. Vorrichtung nach einem der Ansprüche 4 bis 8, d a d u r c h g e k e n n z e i c h n e t , dass der Einsatz (28) mit den Körben (32, 33) von starren Gittern gebildet ist.

9. Vorrichtung nach einem der Ansprüche 4 bis 8, d a d u r c h g e k e n n z e i c h n e t , dass der Boden jedes Korbes konisch zuläuft.

10. Vorrichtung nach einem der Ansprüche 4 bis 9, d a d u r c h g e k e n n z e i c h n e t , dass der Einsatz aus einem einstückigen Kunststoffgussteil besteht.

11. Vorrichtung nach Anspruch 1, d a d u r c h

g e k e n n z e i c h n e t , dass die Einrichtung zur Erzeugung der Schwingungen des Lösungsbades umfasst ein Steuerelement zum manuellen Einstellen des Schwingungsgebers, eine Zeitschalteinrichtung, einen Taktoszillator, einen Ultraschalloszillator, eine Endstufe, einen zur Anpassung dienenden Übertrager und die Ultraschallschwinger-/Wanneneinheit.

1/3

45    46    47

8

T1    2    T2

1

26

**Lenso** *Clean*

FIG 1A

27
4
5

26

6

7    37

FIG 1B

45    46    47

3

FIG 1C

0031152

2l3

FIG 2

FIG 3

FIG 4

FIG 5

3/3

0031152